# EUROPEAN PATENT APPLICATION

(11) **EP 3 111 787 A1**
(43) Date of publication of application: **04.01.2017**
(21) Application number: 15174400.0
(22) Date of filing: 29.06.2015
(51) Int. Cl.: A24F 47/00, A61M 11/04

(54) **ELECTRONIC NICOTINE DELIVERY APPARATUS**

(71) Applicant: JAC Vapour Limited, Edinburgh Lothian EH6 6BZ (GB)
(72) Inventor: LOGAN, Andrew, Edinburgh, Lothian EH6 6BZ (GB); MCGLADE, Euan, Edinburgh, Lothian EH6 6BZ (GB)
(74) Representative: Peter, Kenneth William

(57) **Abstract**

The present invention relates to electronic nicotine delivery apparatus 10. The electronic nicotine delivery apparatus 10 comprises a main body 12 configured to be gripped in one hand by a user and comprising an electric battery. The electronic nicotine delivery apparatus 10 further comprises a delivery arrangement 14 comprising a vaporiser which is electrically powered by the electric battery, the delivery arrangement being releasably attached to the main body 12. The electronic nicotine delivery apparatus 10 yet further comprises a mouthpiece 16 in fluid communication with the delivery arrangement 14. The electronic nicotine delivery apparatus 10 is configured to store e-liquid which is vaporised by the vaporiser during use of the electronic nicotine delivery apparatus. At least one of the main body 12 and the delivery arrangement 14 is configured such that a longitudinal axis of the delivery arrangement is at an angle of at least 5 degrees to a longitudinal axis of the main body.

## Description

### Field of the Invention

The present invention relates to electronic nicotine delivery apparatus and in particular but not exclusively personal electronic nicotine delivery apparatus comprising a main body or box mod, a delivery arrangement or tank and a mouthpiece.

### Background Art

An electronic cigarette is a battery powered vaporiser which is operative to provide a flavour and feel similar to tobacco smoking. An electronic cigarette typically comprises a reservoir, an atomiser, a mouthpiece and power and control apparatus. The reservoir or tank contains e-liquid which typically comprises a mixture of propylene glycol, glycerine, water, nicotine and flavourings. The atomiser is contained within a vaporisation chamber and comprises a small heating element and wicking material. The wicking material is operative to draw e-liquid onto the heating element and the heating element is operative to vaporise drawn e-liquid. Vaporised e-liquid is contained within the vaporisation chamber. The mouthpiece is in fluid communication with the vaporisation chamber whereby a user may draw vaporised e-liquid from the vaporisation chamber. The power and control apparatus comprises at least one battery and user controls.

Design of electronic cigarettes has evolved to so-called third generation devices which are less like tobacco cigarettes in form than earlier devices and are more sophisticated in function. Third generation devices are usually either cylindrical or box shaped. More recent designs of electronic cigarette comprise a main body or box mod, a delivery arrangement or tank and a mouthpiece. The main body contains a battery and control apparatus. The delivery arrangement comprises a reservoir for e-liquid, an atomiser and a vaporisation chamber. Typically the delivery arrangement is releasably connected at one end to the main body and connected to the mouthpiece at the opposite end. Some forms of electronic cigarette are configured to allow for change of the orientation of the mouthpiece in relation to the delivery arrangement.

The present inventors have become appreciative of shortcomings of known electronic cigarettes. The present invention has been devised in light of the inventors' appreciation. It is therefore an object for the present invention to provide improved electronic nicotine delivery apparatus. It is a further object for the present invention to provide improved electronic nicotine delivery apparatus comprising a main body or box mod, a delivery arrangement or tank and a mouthpiece.

### Statement of Invention

According to a first aspect of the present invention there is provided electronic nicotine delivery apparatus comprising:
a main body configured to be gripped in one hand by a user and comprising an electric battery;
a delivery arrangement comprising a vaporiser which is electrically powered by the electric battery, the delivery arrangement being releasably attached to the main body; and
a mouthpiece in fluid communication with the delivery arrangement,
the electronic nicotine delivery apparatus being configured to store e-liquid which is vaporised by the vaporiser during use of the electronic nicotine delivery apparatus, at least one of the main body and the delivery arrangement being configured such that a longitudinal axis of the delivery arrangement is at an angle of at least 5 degrees to a longitudinal axis of the main body.

Electronic nicotine delivery apparatus according to the present invention comprises a main body, a delivery arrangement and a mouthpiece. The main body is configured to be gripped in one hand by a user and comprises an electric battery. The delivery arrangement is releasably attached to the main body. Furthermore the delivery arrangement comprises a vaporiser which is electrically powered by the electric battery when so attached. The mouthpiece is in fluid communication with the delivery arrangement. The electronic nicotine delivery apparatus is configured to store e-liquid, such as in at least one of the delivery arrangement and the main body. In use, e-liquid is vaporised by the vaporiser and the user draws vaporised e-liquid into his or her mouth by way of the mouthpiece. The present inventors have found that configuring at least one of the main body and the delivery arrangement such that a longitudinal axis of the delivery arrangement is at an angle of at least 5 degrees to a longitudinal axis of the main body provides for ease of use. More specifically such angulation of the delivery arrangement in relation to the main body has been found to present the mouthpiece to the user in a convenient manner whilst the main body is gripped by the user. In contrast, in some known electronic nicotine delivery apparatus the main body, the delivery arrangement and the mouthpiece are disposed in relation to each other along the same longitudinal axis. In other known electronic nicotine delivery apparatus the mouthpiece and the delivery arrangement are configured for change of orientation of the mouthpiece in relation to the delivery arrangement. Nevertheless, the present inventors have found the present invention to provide for improved ease of use over such latter known electronic nicotine delivery apparatus.

The present inventors have found that an angle of between 5 degrees and 30 degrees may provide for reasonable ease of use. The present inventors have found that an angle of between 7 degrees and 17 degrees may provide for improved ease of use. At least one of the main body and the delivery arrangement may be configured such that a longitudinal axis of the delivery arrangement is at an angle of at least 5 degrees, 7 degrees, 9 degrees, 10 degrees, 11 degrees, 12 degrees, 13 degrees, 14 degrees, 15 degrees, 16 degrees, 18 degrees, 20 degrees, 22 degrees, 24 degrees, 26 degrees or 28 degrees to a longitudinal axis of the main body. Alternatively or in addition, at least one of the main body and the delivery arrangement may be configured such that a longitudinal axis of the delivery arrangement is at an angle of no more than 30 degrees, 28 degrees, 26 degrees, 24 degrees, 22 degrees, 20 degrees, 18 degrees, 16 degrees, 15 degrees, 14 degrees, 13 degrees, 12 degrees, 11 degrees, 10 degrees, 9 degrees or 7 degrees to a longitudinal axis of the main body. The present inventors have found that under certain circumstances an angle of between 10 degrees and 14 degrees and more specifically an angle of 12 degrees may provide for superior ease of use.

The electronic nicotine delivery apparatus may comprise a connector arrangement which is configured to provide for releasable connection of the delivery arrangement to the main body. The connector arrangement may comprise a first connector and a second connector, the first connector and the second connector having inter-engaging profiles. For example, the first and second connectors may provide for connection to each other by way of a bayonet arrangement. In a preferred form, the first and second connectors may be configured for connection to each other by way of threaded profiles. By way of a first example the connector arrangement may comprise a 510 connector arrangement. By way of a second example the connector arrangement may comprise a KR808 connector arrangement. By way of a third example the connector arrangement may comprise an Eagle connector arrangement.

The first connector may be comprised in the main body and the second connector may be comprised in the delivery arrangement. The first connector may be comprised in an end of the main body. The second connector may be comprised in an end of the delivery arrangement. The electronic nicotine delivery apparatus may be configured such that one of the first and second connectors may be oriented at an angle to the longitudinal axis of the part in which it is comprised. More specifically the first connector may be oriented at an angle to the longitudinal axis of the main body. The first connector may be oriented at an angle of at least 5 degrees to the longitudinal axis of the main body. Angulation of the delivery arrangement in relation to the main body may be achieved solely by way of orientation of the first and second connectors relative to a respective part in which each is comprised. Features described hereinabove in respect of extent of angulation may therefore apply here in respect of extent of angulation of a connector.

The main body may comprise a housing. The housing may be cylindrical in form. Alternatively the housing may have the general form of a rectangular cuboid. Alternatively a cross-section through the housing may be oval with one axis of symmetry. The housing may be elongate and more specifically of sufficient length to be gripped in one hand. The housing may contain an electric battery, such as a rechargeable electric battery. The housing may comprise user controls, such as an on-off switch and power level control. The housing may comprise at least one display which is operative, for example, to provide visual indication when the electronic nicotine delivery apparatus is switched on, of battery level and of power level.

The housing may define an end of the main body and a side of the main body. The end may define a surface which is at an angle to a side of the main body. The first connector may be mounted in the end of the main body. The first connector may be set at the requisite angle thereby.

The housing may be between 8 cm and 9 cm long and more specifically 8.5 cm long. The housing may be between 3cm and 3.5 cm across its greatest width and more specifically 3.3 cm wide.

The delivery arrangement may define a chamber. The chamber may contain the vaporiser. The chamber may constitute a vaporisation chamber, such as in respect of part of the chamber towards the mouthpiece when the chamber contains the vaporiser.

The chamber may be configured to contain e-liquid. More specifically the chamber may be configured such that e-liquid is contained in the chamber towards the main body when the electronic nicotine delivery apparatus is in use. A side of the chamber or part thereof may be transparent or translucent whereby a level of e-fluid in the chamber may be visually determined. The electronic nicotine delivery apparatus may further comprise a wicking arrangement. The wicking arrangement may be configured to draw e-liquid onto the vaporiser. The wicking arrangement may be contained in the chamber. More specifically the wicking arrangement may be disposed in the chamber relative to the vaporiser whereby e-liquid is drawn onto the vaporiser from e-liquid contained within the chamber.

According to a second aspect of the present invention there is provided electronic nicotine delivery apparatus comprising:
a main body comprising an electric battery;
a delivery arrangement comprising a vaporiser which is electrically powered by the electric battery, the delivery arrangement being attached to the main body; and
a mouthpiece in fluid communication with the delivery arrangement.

The electronic nicotine delivery apparatus may be personal electronic nicotine delivery apparatus. The electronic nicotine delivery apparatus may be configured to be hand-held. The main body may be configured to be gripped in one hand by a user. The main body may define a side around which a user's hand extends when gripping the main body during use of the electronic nicotine delivery apparatus. The side of the main body may extend in a first direction. The delivery arrangement may define a side between an end which is oriented towards the main body and an end from which the mouthpiece extends. The end of the delivery arrangement which is oriented towards the main body may extend in a second direction. The second direction may be at an angle of at least 95 degrees to the first direction.

Further embodiments of the second aspect of the present invention may comprise one or more features of the first aspect of the present invention and vice-versa.

### Brief Description of Drawings

Further features and advantages of the present invention will become apparent from the following specific description, which is given by way of example only and with reference to the accompanying drawings, in which:
Figure 1A is a view from one side of an embodiment of the electronic nicotine delivery apparatus;
Figure 1B is a view from the front of the embodiment of Figure 1A;
Figure 2A is a perspective view from the side and above of the main body of the embodiment of Figure 1 A; and
Figure 2B is a view from above of the end of the main body of the embodiment of Figure 1A.

### Description of Embodiments

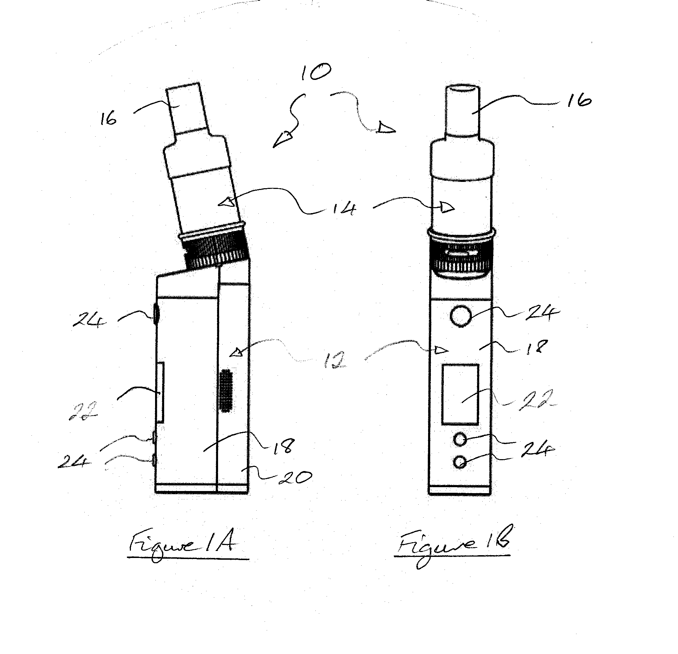

A view from one side of an embodiment of the electronic nicotine delivery apparatus 10 is shown in Figure 1A. A view from the front of the embodiment of Figure 1A is shown in Figure 1B. The electronic nicotine delivery apparatus 10 comprises a main body 12, a delivery arrangement 14 and a mouthpiece 16. The delivery arrangement 14 is releasably connected at one end to the main body and connected at the other end to the mouthpiece 16. The main body 12 comprises a housing 18 which contains a re-chargeable electric battery (not shown) and electronic apparatus for control and display purposes. As can be seen from Figure 1A the housing comprises a removable cover 20 whereby access may be gained to the electric battery. The housing 18 also supports an LCD display 22 which is operative to provide visual indication of the electronic nicotine delivery apparatus being switched on, battery charge level and presently set power level for the vaporiser. In addition the housing 18 supports user operable controls 24 to switch the electronic nicotine delivery apparatus 10 on and off and to set the power for the vaporiser at a desired level. The housing is 8.443 cm long and 3.3 cm across its greatest width.

The delivery arrangement 14 has a chamber formed from stainless steel with a glass window formed in the side thereof. The chamber contains a vaporiser (not shown) and a wicking arrangement (not shown). The wicking arrangement is disposed towards the end of the chamber closer to the main body with the vaporiser being disposed above the wicking arrangement. The chamber is configured to hold a quantity of e-fluid with the e-fluid being contained in the part of the chamber closer to the main body when the electronic nicotine delivery apparatus 10 is held upright during use. The delivery arrangement 14 in respect of form and function is known. Considering operation of the electronic nicotine delivery apparatus 10, the e-fluid held in the part of the chamber closer to the main body is drawn by the wicking arrangement onto the vaporiser where it is vaporised. The vaporised e-fluid is then present in the part of the chamber closer to the mouthpiece 16 wherefrom it may be drawn by the user through the mouthpiece as the user grips the housing 18 in one hand. The part of the chamber closer to the mouthpiece 16 therefore constitutes a vaporisation chamber.

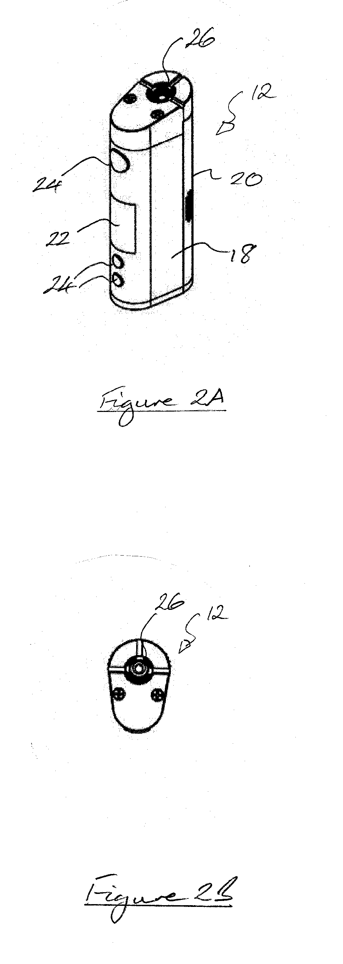

A perspective view from the side and above of the main body of the embodiment of Figure 1A is shown in Figure 2A and a view from above of the end of the main body of the embodiment of Figure 1A is shown in Figure 2B. The main body 12 and the delivery arrangement 14 are releasably connected to each other by way of a 510 connector arrangement. A first part 26 of the 510 connector arrangement is comprised in an end of the main body 12 and a second part of the 510 connector arrangement is comprised in the end of the delivery arrangement 14 opposite the end at which the mouthpiece 16 is connected. The first and second parts of the 510 connector threadedly engage with each other in accordance with known practice for this form of connector. As can be seen from Figure 2A and Figure 1A in particular, the end of the housing 18 of the main body 12 comprising the first part 26 of the 510 connector arrangement is at an angle to the sides of the housing 18. The end of the housing 18 of the main body 12 comprising the first part 26 of the 510 connector arrangement is therefore at an angle to a longitudinal axis of the main body, i.e. an axis running lengthwise along the main body parallel to the sides of the housing. In the embodiment of Figures 1A to 2B, the end of the housing 18 of the main body 12 comprising the first part 26 of the 510 connector arrangement is at an angle of 12 degrees to the sides of the housing 18. Hence the first part 26 of the 510 connector arrangement is at a corresponding angle whereby the delivery arrangement 14 is at a corresponding angle to the sides of the housing 18 when attached by way of the 510 connector arrangement. Such an angle has been found to present the mouthpiece 16 to the user in a convenient manner whilst the main body 12 is gripped by the user.

## Claims

1. Electronic nicotine delivery apparatus comprising:
a main body configured to be gripped in one hand by a user and comprising an electric battery;
a delivery arrangement comprising a vaporiser which is electrically powered by the electric battery, the delivery arrangement being releasably attached to the main body; and
a mouthpiece in fluid communication with the delivery arrangement,
the electronic nicotine delivery apparatus being configured to store e-liquid which is vaporised by the vaporiser during use of the electronic nicotine delivery apparatus, at least one of the main body and the delivery arrangement being configured such that a longitudinal axis of the delivery arrangement is at an angle of at least 5 degrees to a longitudinal axis of the main body.

2. Electronic nicotine delivery apparatus according to claim 1 in which the longitudinal axis of the delivery arrangement is at an angle of between 5 degrees and 30 degrees to the longitudinal axis of the main body.

3. Electronic nicotine delivery apparatus according to claim 1 in which the longitudinal axis of the delivery arrangement is at an angle of between 7 degrees and 17 degrees to the longitudinal axis of the main body.

4. Electronic nicotine delivery apparatus according to any one of the preceding claims comprising a connector arrangement which is configured to provide for releasable connection of the delivery arrangement to the main body, the connector arrangement comprising a first connector and a second connector, the first connector and the second connector having inter-engaging profiles.

5. Electronic nicotine delivery apparatus according to claim 4 in which the first connector is comprised in an end of the main body and the second connector is comprised in an end of the delivery arrangement, the electronic nicotine delivery apparatus being configured such that one of the first and second connectors is oriented at an angle to the longitudinal axis of the part in which it is comprised.

6. Electronic nicotine delivery apparatus according to claim 5 in which the first connector is oriented at an angle to the longitudinal axis of the main body.

7. Electronic nicotine delivery apparatus according to any one of the preceding claims in which the main body comprises a housing.

8. Electronic nicotine delivery apparatus according to claim 7 in which a cross-section through the housing is oval with one axis of symmetry.

9. Electronic nicotine delivery apparatus according to claim 7 or 8 in which the housing contains an electric battery and comprises user controls.

10. Electronic nicotine delivery apparatus according to any one of claims 7 to 9 in which the housing defines an end of the main body and a side of the main body, the end of the main body defining a surface which is at an angle of at least 95 degrees to the side of the main body.

11. Electronic nicotine delivery apparatus according to any one of the preceding claims in which the delivery arrangement defines a chamber.

12. Electronic nicotine delivery apparatus according to claim 11 in which the chamber contains the vaporiser.

13. Electronic nicotine delivery apparatus according to claim 11 or 12 in which the chamber is configured to contain e-liquid.

14. Electronic nicotine delivery apparatus according to any one of the preceding claims further comprising a wicking arrangement which is configured to draw e-liquid onto the vaporiser.

15. Electronic nicotine delivery apparatus according to claim 14 in which the delivery arrangement defines a chamber and the wicking arrangement is supported in the chamber relative to the vaporiser whereby e-liquid is drawn onto the vaporiser from e-liquid contained within the chamber.
